# EUROPÄISCHE PATENTANMELDUNG

(11) **EP 0 752 410 A2**
(43) Veröffentlichungstag der Anmeldung: **08.01.1997**
(21) Anmeldenummer: 96110197.9
(22) Anmeldetag: 25.06.1996
(51) Int. Cl.: C07C 209/60

(54) **Verfahren zur Herstellung von Aminen aus Olefinen an übergangsmetallfreien, ohne Halogenbehandlung hergestellten Aluminium-BETA-Zeolithen**

(30) Priorität: 04.07.1995 DE 19524241
(71) Anmelder: BASF AKTIENGESELLSCHAFT, 67056 Ludwigshafen (DE)
(72) Erfinder: Dingerdissen, Uwe, Dr., 64342 Seeheim-Jugenheim (DE); Kummer, Rudolf, Dr., 67227 Frankenthal (DE); Stops, Peter, 67122 Altrip (DE); Herrmann, Jürgen, 68307 Mannheim (DE); Lützel, Hans-Jürgen, 67459 Böhl-Iggelheim (DE); Eller, Karsten, Dr., 67059 Ludwigshafen (DE)

(57) **Zusammenfassung**

Verfahren zur Herstellung von Aminen der allgemeinen Formel I 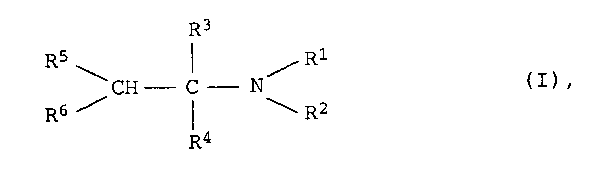 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃- bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 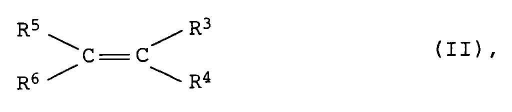 in der R³, R⁴, R⁵ und R⁶ die oben genannten Bedeutungen haben, mit Ammoniak oder primären oder sekundären Aminen der allgemeinen Formel III 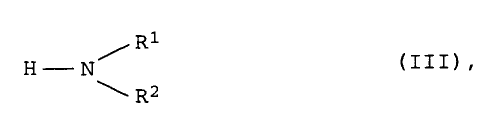 in der R¹ und R² die oben genannten Bedeutungen haben, bei Temperaturen von 250 bis 350°C und Drücken von 150 bis 300 bar in Gegenwart eines zeolithischen Katalysators, indem man als zeolithischen Katalysator übergangsmetallfreie Aluminium-BETA-Zeolithe einsetzt, die ohne Halogenbehandlung hergestellt wurden.

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart von übergangsmetallfreien, ohne Halogenbehandlung hergestellten Aluminium-BETA-Zeolithen.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Nachteilig sind die dort mit dieser Zeolith-Klasse erreichten Ausbeuten von weniger als 14 % bei geringen Belastungen und hohem Ammoniak-Überschuß.

Der vorliegenden Erfindung lag daher die Aufgabe zugrunde, den zuvor genannten Nachteilen abzuhelfen und insbesondere zu höheren Raum-Zeit-Ausbeuten zu gelangen.

Demgemäß wurde ein neues und verbessertes Verfahren zur Herstellung von Aminen der allgemeinen Formel I 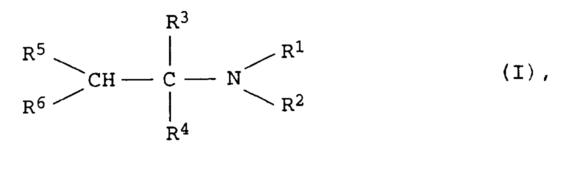 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃- bis C₉-Alkylendikette,
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 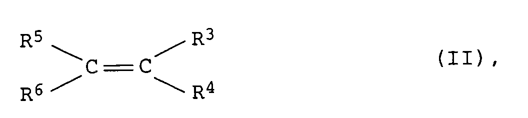 in der R³, R⁴, R⁵ und R⁶ die oben genannten Bedeutungen haben, mit Ammoniak oder primären oder sekundären Aminen der allgemeinen Formel III 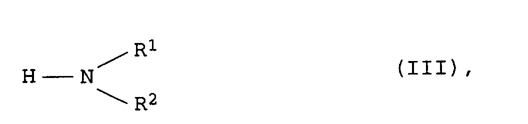 in der R¹ und R² die oben genannten Bedeutungen haben, bei Temperaturen von 250 bis 350°C und Drücken von 150 bis 300 bar in Gegenwart eines zeolithischen Katalysators gefunden, welches dadurch gekennzeichnet ist, daß man als zeolithischen Katalysator übergangsmetallfreie Aluminium-BETA-Zeolithe einsetzt, die ohne Halogenbehandlung hergestellt wurden.

Das erfindungsgemäße Verfahren kann wie folgt durchgeführt werden:

Das Olefin II und Ammoniak oder das primäre oder sekundären Amin III kann bei Temperaturen von 250 bis 350°C, bevorzugt 260 bis 330°C, besonders bevorzugt 260 bis 320°C und Drücken von 150 bis 300 bar, bevorzugt 170 bis 300 bar, besonders bevorzugt 200 bis 300 bar in Gegenwart eines übergangsmetallfreien Aluminium-BETA-Zeoliths, der ohne Halogenbehandlung hergestellt wurde, als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung der Katalysatoren zurückgedrängt worden.

Das erfindungsgemäße Verfahren zeichnet sich dadurch aus, daß bereits mit niedrigem Ammoniak- bzw. Amin-Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 150 bis 300 bar und einer Temperatur von 250 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Die erfindungsgemäßen Aluminium-BETA-Zeolithe werden weder verformt noch unverformt einer Behandlung mit halogenhaltigen Reagenzien, wie Salzsäure (HCl), Flußsäure (HF) oder Ammoniumfluorid (NH₄F) unterworfen. Ferner enthalten die erfindungsgemäßen Aluminium-BETA-Zeolithe keine Übergangsmetalle der Gruppen IB, VB, VIB, VIIB und VIII des Periodensystems der Elemente wie Chrom, Mangan, Eisen, Nickel, Palladium, Platin oder Kupfer.

Als Katalysatoren für die Aminierung von Olefinen eignen sich übergangsmetallfreie Aluminium-BETA-Zeolithe oder auch NU-1-Zeolithe, die ohne Halogenbehandlung hergestellt wurden, wie sie beispielsweise in US-A 3,308,069 oder US-A-4,060,590 beschrieben sind.

Die übergangsmetallfreien, ohne Halogenbehandlung hergestellten Aluminium-BETA-Zeolithe, die bevorzugt in der H-Form eingesetzt werden, werden in der Regel mit einem Bindemittel im Verhältnis 98:2 bis 40:60 Gew.-% zu Strängen oder Tabletten verformt. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 11O°C/16 h getrocknet und bei 300-500°C/2-16 h calciniert, wobei die Calcinierung auch direkt im Aminierungsreaktion erfolgen kann.

Zur Erhöhung der Selektivität, der Standzeit und der Anzahl der möglichen Regenerierungen kann man verschiedene Modifizierungen an zeolithischen Katalysatoren vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl und seltenen Erdmetallen wie z.B. La, Ce oder Y ionenaustauschen bzw. dotieren kann.

Eine vorteilhafte Ausführungsform besteht darin, daß man die verformten übergangsmetallfreien, ohne Halogenbehandlung hergestellten Aluminium-BETA-Zeolithe in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der Zeolithe vorgenommen werden.

Eine andere Möglichkeit der Metallaufbringung auf die Zeolithe besteht darin, daß man das zeolithische Material z.B. mit einem Halogenid, einem Acetat, einem Oxalat, einem Citrat, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen kann eine Nachbehandlung mit Wasserstoff und/oder mit Wasserdampf günstig sein.

Eine andere Möglichkeit der Modifizierung ist gegeben durch einen Austausch mit Ammonsalzen, z.B. mit NH₄NO₃ oder mit Mono-, Di- oder Polyaminen. Hierbei wird der mit Bindemittel verformte Zeolith in der Regel bei 60 bis 80°C mit 10 bis 25%iger, bevorzugt 20%iger NH₄NO₃-Lösung 2 h kontinuierlich in gewichtsmäßiger Zeolith/Ammonnitrat-Lösung von 1:15 ausgetauscht und danach bei 100 bis 120°C getrocknet.

Eine weitere Möglichkeit der Modifikation besteht darin, daß man die Zeolithe - verformt oder unverformt - einer Behandlung mit halogenfreien Säuren, wie Schwefelsäure (H₂SO₄), Phosphorsäure (H₃PO₄) oder Oxalsäure (HO₂C-CO₂H) unterwirft.

Darüber hinaus kann an den erfindungsgemäßen Zeolithen auch eine Dealuminierung vorgenommen werden, bei der die Zeolithe z.B. durch eine hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₂₀-Alkenyl, bevorzugt C₂- bis C₁₂-Alkenyl, besonders bevorzugt C₂- bis C₈-Alkenyl wie Vinyl und Allyl,
   - C₂- bis C₂₀-Alkinyl, bevorzugt C₂- bis C₈-Alkinyl, besonders bevorzugt C₂H und Propargyl,
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
   - C₄- bis C₂₀-Cycloalkyl-alkyl, bevorzugt C₄- bis C₁₂-Cycloalkyl-alkyl, besonders bevorzugt C₅- bis C₁₀-Cycloalkyl-alkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Nethylphenyl, 3-Methylphenyl, 4-Methylphenyl, 2-Ethylphenyl, 3-Ethylphenyl, 4-Ethylphenyl und
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl und
R¹ und R²
   - gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ oder R⁵
   - C₂₁- bis C₂₀₀-Alkyl, bevorzugt C₄₀- bis C₂₀₀-Alkyl wie Polybutyl, Polyisobutyl, Polypropyl, Polyisopropyl und Polyethyl, besonders bevorzugt Polybutyl und Polyisobutyl,
   - C₂₁- bis C₂₀₀-Alkenyl, bevorzugt C₄₀- bis C₂₀₀-Alkenyl, besonders bevorzugt C₇₀- bis C₁₇₀-Alkenyl,
R³ oder R⁵
   - gemeinsam eine C₂- bis C₁₂-Alkylendikette, bevorzugt eine C₃- bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysator A

150g Beta-Zeolith (Fa. Degussa) wurden mit 7,5g Aerosil 200 (Fa.Degussa), 12g NH₄OH 25% und 7,5% Stärke versetzt. Im Kneter wurde die Mischung kompaktiert und unter vorsichtigem Wasser-zusatz (max. 125 ml) verknetet. Die Knetzeit betrug 60 min. In einer Strangpresse wurden mit einem Preßdruck von 120 bar 2mm Stränge erzeugt, 16 h bei 110°C getrocknet und 16 h bei 500°C kalziniert.

### Katalysator B

60g Beta-Zeolith (Fa. Uetikon) wurden mit 40g Boehmit und 2g Ameisensäure versetzt. Im Kneter wurde die Mischung kompaktiert und unter vorsichtigem Wasserzusatz (max. 65 ml) verknetet. Die Knetzeit betrug 60 min. In einer Strangpresse wurden mit einem Preßdruck von 80 bar 2mm Stränge erzeugt, 16 h bei 120°C getrocknet und 16 h bei 500°C kalziniert.

### Katalysator C

60g Beta-Zeolith (Fa. Degussa) wurden mit 40g Boehmit und 2g Ameisensäure versetzt. Im Kneter wurde die Mischung kompaktiert und unter vorsichtigem Wasserzusatz (max. 64 ml) verknetet. Die Knetzeit betrug 40 min. In einer Strangpresse wurden mit einem Preßdruck von 55 bar 2mm Stränge erzeugt, 16 h bei 120°C getrocknet und 16 h bei 500°C kalziniert.

### Katalysator D

100 g Beta-Zeolith (Fa. Degussa) wurden mit 6,7 g Kieselsol (AS40, Fa.Bayer), 8 g NH₄OH 25% und 5g Stärke versetzt. Im Kneter wurde die Mischung kompaktiert und unter vorsichtigem Wasserzusatz (82 ml) verknetet. Die Knetzeit betrug 50 min. In einer Strangpresse wurden mit einem Preßdruck von 80 bar 2mm Stränge erzeugt, 4 h bei 120°C getrocknet und 16 h bei 500°C kalziniert.

### Katalysator E

100 g Beta-Zeolith (Fa. Degussa) wurden mit 10 g Aerosil® 200 (Fa. Degussa) und 5,5 g Stärke versetzt. Im Kneter wurde die Mischung kompaktiert und unter vorsichtigem Wasserzusatz (110 ml) verknetet. Die Knetzeit betrug 40 min. In einer Strangpresse wurden mit einem Preßdruck von 75 bar 2mm Stränge erzeugt, 4 h bei 110°C getrocknet und 16 h bei 500°C kalziniert.

### Katalysator F

180 g Beta-Zeolith (Fa. Uetikon) wurden mit 120 g Boehmit und 6 g Ameisensäure versetzt. Im Kneter wurde die Mischung kompaktiert und unter vorsichtigem Wasserzusatz (210 ml) verknetet. Die Knetzeit betrug 45 min. In einer Strangpresse wurden mit einem Preßdruck von 70 bar 2mm Stränge erzeugt, 4 h bei 110°C getrocknet und 16 h bei 500°C kalziniert.

### Katalysator G

480 g Beta-Zeolith (Fa. Uetikon) wurden mit 120 g Boehmit und 12 g Ameisensäure versetzt. Im Kneter wurde die Mischung kompaktiert und unter vorsichtigem Wasserzusatz (500 ml) verknetet. Die Knetzeit betrug 45 min. In einer Strangpresse wurden mit einem Preßdruck von 80 bar 2mm Stränge erzeugt, 4 h bei 110°C getrocknet und 16 h bei 500°C kalziniert.

### Vergleichsbeispiele (dotierte BETA-Zeolithe)

### Katalysator H

17 g Katalysator B in Splittform wurden in einem 500 ml Kolben vorgelegt und eine Lösung aus 10ml Wasser und 0,5g NH₄F zugegeben. Nach einer halben Stunde wurde das Wasser bei 80°C (15mbar) abgezogen. Der Katalysator wurde 16 h bei 120°C getrocknet.

### Katalysator I

100g Beta-Zeolith (Fa. Degussa) in Pulverform wurden 3g NH₄F in 100 ml Wasser behandelt. Nach einer halben Stunde wurde das Wasser bei 80°C (15mbar) abgezogen. Der Katalysator wurde 14 h bei 110°C getrocknet.

60 g dieses Katalysators wurden mit 40g Boehmit und 2g Ameisensäure versetzt. Im Kneter wurde die Mischung kompaktiert und unter Wasserzusatz (65 ml) verknetet. Die Knetzeit betrug 45 min. In einer Strangpresse wurden mit einem Preßdruck von 80 bar 2mm Stränge erzeugt, 4 h bei 110°C getrocknet und 16 h bei 500°C kalziniert.

### Katalysator J

Zu 19 g Katalysator B wurde eine Lösung aus 20 ml Wasser, 1,9 g Oxalsäure und 0,1 g Pd-Acetat gegeben. Nach 30 min wurde das Wasser bei 80°C (15mbar) abgezogen. Der Katalysator wurde 16 h bei 120°C getrocknet und 16 h bei 500°C kalziniert.

### Katalysator K

Zu 20 g Katalysator I wurde eine Lösung aus 20 ml Wasser, 2 g Oxalsäure und 0,1 g Pd-Acetat zugegeben. Nach 0,5 Stunden wurde das Wasser bei 80°C (15mbar) abgezogen. Der Katalysator wurde 16 h bei 120°C getrocknet und 16 h bei 500°C kalziniert.

### Vergleichsbeispiele 2 (andere Zeolithe)

### Katalysator L

525 g H-Y-Zeolith (BASF) wurden mit 225 g Boehmit und 15 g Ameisensäure versetzt. Im Kneter wurde die Mischung kompaktiert und unter vorsichtigem Wasserzusatz (590 ml) verknetet. Die Knetzeit betrug 45 min. In einer Strangpresse wurden mit einem Preßdruck von 90 bar 2mm Stränge erzeugt, 16 h bei 120°C getrocknet und 16 h bei 500°C kalziniert.

### Katalysator M

60g ZSM-5-Zeolith (Fa.Vereinigte Aluminium Werke) wurden mit 40g Boehmit und 2g Ameisensäure versetzt. Im Kneter wurde die Mischung kompaktiert und unter vorsichtigem Wasserzusatz (55 ml) verknetet. Die Knetzeit betrug 40 min. In einer Strangpresse wurden mit einem Preßdruck von 55 bar 2mm Stränge erzeugt, 2 h bei 120°C getrocknet und 16 h bei 400°C kalziniert.

### Aminierungsbeispiele

In einen 0,3 l-Rührautoklaven wurden jeweils 10 ml der oben beschriebenen Katalysatoren gegeben und nach dem Verschließen die Olefine und Ammoniak aufgedrückt. Die Menge an Einsatzprodukt wurde so bemessen, daß bei der gewählten Reaktionstemperatur der gewünschte Druck als Eigendruck der Reaktionspartner erreicht wurde.

Der Austrag wurde getrennt nach Flüssig- und Gasphase gaschromatographisch untersucht. Die in den Tabellen aufgeführten Umsätze beziehen sich immer auf das Olefin; die angegebenen Ausbeuten beziehen sich auf die Hauptprodukte: Cyclohexylamin aus Cyclohexen, Cyclopentylamin aus Cyclopenten, tert.-Butylamin aus Isobuten.

Für eine kontinuierliche Herstellung wurde ein Hochdruck-Reaktor mit 2 m Länge und 24 mm Innendurchmesser eingesetzt, der mittels einer Alublockheizung beheizt und mit dreifacher Innentemperaturmessung sowie mit einer Druckhaltung ausgestattet ist. Es wurden jeweils 60 ml Katalysator eingebaut und der obere Teil des Reaktorrohres mit Porzellanringen gefüllt. Der Zulauf von Olefin und Ammoniak erfolgte von oben.

Die Analyse der Reaktorausträge wurde gaschromatographisch und wahlweise zusätzlich destillativ durchgeführt.

Wahlweise wurden die Versuche in einem Rohrreaktor (6 mm Innendurchmesser) unter isothermen Bedingungen bei 270 bis 340°C und einem Druck von 280 bar mit einem Gemisch aus Olefinen und Ammoniak im molaren Verhältnis von 1:1,5 bis 1:2 durchgeführt. Die Reaktionsprodukte wurden im Gaschromatographen analysiert.

Die Ergebnisse mit den verschiedenen Katalysatoren sind in den Tabellen 1 bis 4 zusammengestellt.

**Tabelle 1**

| tert.-Butylamin (NH₃: C₄H₈ = 1,5) | | | | | | | | |
|---|---|---|---|---|---|---|---|---|
| Katalysator | Anteil Verstrangungshilfsmittel | | Druck | Temperatur | tert.-Butyl-Amin-Ausbeute [Gew.-%] | | | Litergewicht |
| | Al₂O₃ [%] | SiO₂ [%] | [bar] | [°C] | WHSV 0,7 g/g h | WHSV 1,5 g/g h | WHSV 3 g/g h | [kg/l] |
| A | | 5 | 280 | 270 | 21,71 | 21,2 | 19,15 | 0,42 |
| B | 40 | | 280 | 270 | 21,52 | 19,96 | 16,15 | 0,5 |
| C | 40 | | 280 | 270 | 21,62 | 19,88 | 16,26 | 0,53 |
| D | | 2 | 280 | 270 | 21,15 | 20,42 | 16,38 | 0,45 |
| E | | 9 | 280 | 270 | 22,03 | 20,57 | 19,69 | 0,41 |
| F | 40 | | 280 | 270 | 21,79 | 19,76 | 16,49 | 0,55 |
| G | 20 | | 280 | 270 | 22,48 | 21,08 | 18,38 | 0,49 |
| H | 40 | | 280 | 270 | 21,44 | 19,61 | 16,84 | 0,50 |
| I | 40 | | 280 | 270 | 21,17 | 19,2 | 16,32 | 0,52 |
| J | 40 | | 280 | 270 | 21,53 | 18,79 | 16,11 | 0,58 |
| K | 40 | | 280 | 270 | 20,79 | 18,58 | 15,82 | 0,55 |
| L | 30 | | 280 | 270 | 20,58 | 13,54 | 8,6 | 0,49 |
| M | 40 | | 280 | 270 | 20,44 | 17,77 | 13,69 | 0,55 |

Es zeigte sich, daß eine Behandlung der Katalysatoren mit Palladium und/oder Fluor nicht zu einer Verbesserung führte. In einigen Fällen ließ sich sogar eine Verschlechterung feststellen. Im Falle einer Pd-Behandlung kam es sogar zu einer nachteiligen Bildung von Polymeren. Ebenso wiesen die Vergleichskatalysatoren I,J eine geringere Raum-Zeit-Ausbeute auf.

**Tabelle 2**

| Vergleichsbeispiele nach CA 2 092 964, Bsp. I tert.-Butylamin (NH₃ / C₄H₈ = 2) | | | | | | |
|---|---|---|---|---|---|---|
| Kat. | SiO₂ [%] | bar | °C | WHSV [g/g·h] | Umsatz IB [%] | Ausbeute TBA [mol.-%] |
| A | 5 | 100 | 280 | 1,0 | 11,29 | 9,84 |
| A | 5 | 100 | 300 | 1,0 | 15,24 | 6,20 |

Das Beispiel zeigt, daß bei niedrigen Drücken und Belastungen nur geringe Umsätze bei schlechtem Selektivitäten erzielt werden.

**Tabelle 3**

| Cyclopentylamin (NH₃ / C₅H₈ = 2) | | | | | | | | |
|---|---|---|---|---|---|---|---|---|
| Katalysator | Anteil Verstrangungshilfsmittel | | Druck | Temperatur | Cyclopentylamin-Ausbeute [Gew.-%] | | | Litergewicht |
| | Al₂O₃ [%] | SiO₂ [%] | [bar] | [°C] | WHSV 0,7 g/g·h | WHSV 1,5 g/g·h | WHSV 3 g/g·h | [kg/l] |
| A | | 5 | 280 | 320 | | 10,0 | 10,5 | 0,42 |
| A | | 5 | 280 | 340 | | | 12,3 | 0,42 |

**Tabelle 4**

| Cyclohexylamin (NH₃ / C₆H₁₀ = 1,5) | | | | | |
|---|---|---|---|---|---|
| Katalysator | Druck | Temperatur | Cyclohexylamin-Ausbeute [Mol.-%] | | |
| | [bar] | [°C] | Verweilzeit 16 h | | |
| β-Zeolith (Fa. Ketikon) | 550 | 300 | 7,1 | | |

## Patentansprüche

1. Verfahren zur Herstellung von Aminen der allgemeinen Formel I 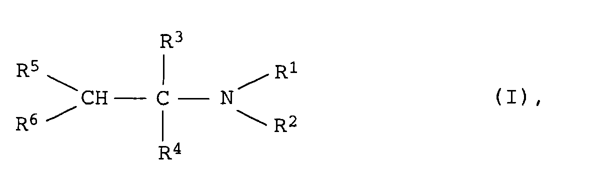 in der
R¹,R²,R³,R⁴,R⁵,R⁶ Wasserstoff, C₁- bis C₂₀-Alkyl, C₂- bis C₂₀-Alkenyl, C₂- bis C₈-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 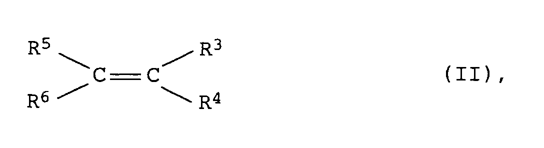 in der R³, R⁴, R⁵ und R⁶ die oben genannten Bedeutungen haben, mit Ammoniak oder primären oder sekundären Aminen der allgemeinen Formel III 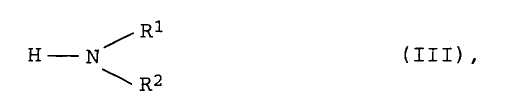 in der R¹ und R² die oben genannten Bedeutungen haben, bei Temperaturen von 250 bis 350°C und Drücken von 150 bis 300 bar in Gegenwart eines zeolithischen Katalysators, dadurch gekennzeichnet, daß man als zeolithischen Katalysator übergangsmetallfreie Aluminium-BETA-Zeolithe einsetzt, die ohne Halogenbehandlung hergestellt wurden.

2. Verfahren zur Herstellung von Aminen I nach Anspruch 1, dadurch gekennzeichnet, daß man Aluminium-BETA-Zeolithe in der H-Form einsetzt.

3. Verfahren zur Herstellung von Aminen I nach den Ansprüchen 1 bis 2, dadurch gekennzeichnet, daß man das gebildete Amin I abtrennt und die nichtumgesetzten Einsatzstoffe II und III zurückführt.

4. Verfahren zur Herstellung von Aminen nach den Ansprüchen 1 bis 3, dadurch gekennzeichnet, daß man als zeolithische Katalysatoren Aluminium-BETA-Zeolithe einsetzt, die mit einem Bindemittel verformt und bei Temperaturen von 300 bis 600°C calciniert sind.

5. Verfahren zur Herstellung von Aminen nach den Ansprüchen 1 bis 4, dadurch gekennzeichnet, daß man als Olefin II Isobuten, Diisobuten, Cyclopenten, Cyclohexen oder Polyisobuten einsetzt.

6. Verfahren zur Herstellung von Aminen nach den Ansprüchen 1 bis 5, dadurch gekennzeichnet, daß man als zeolithischen Katalysatoren Aluminium-BETA-Zeolithe einsetzt, die ohne eine Behandlung mit einem halogenhaltigen Reagenz aus der Gruppe Salzsäure, Flußsäure oder NH₄F hergestellt wurden.

7. Verfahren zur Herstellung von Aminen nach den Ansprüchen 1 bis 6, dadurch gekennzeichnet, daß man als zeolithische Katalysatoren Aluminium-BETA-Zeolithe einsetzt, die undotiert sind.

8. Verfahren zur Herstellung von Aminen nach den Ansprüchen 1 bis 7, dadurch gekennzeichnet, daß man als zeolithische Katalysatoren Aluminium-BETA-Zeolithe, die mit einem oder mehreren Elementen der Seltenen Erden dotiert sind, einsetzt.

9. Verfahren zur Herstellung von Aminen nach den Ansprüchen 1 bis 8, dadurch gekennzeichnet, daß man als zeolithische Katalysatoren Aluminium-BETA-Zeolithe, die mit einem oder mehreren Alkali-, Erdalkali- oder Erdmetallen aus der Gruppe Natrium, Kalium, Calcium, Magnesium und Thallium dotiert sind, einsetzt.

10. Verfahren zur Herstellung von Aminen nach den Ansprüchen 1 bis 9, dadurch gekennzeichnet, daß man als zeolithische Katalysatoren Aluminium-BETA-Zeolithe in der Ammoniumform einsetzt.

11. Verfahren zur Herstellung von Aminen nach den Ansprüchen 1 bis 10, dadurch gekennzeichnet, daß man als zeolitische Katalysatoren Aluminium-BETA-Zeolithe einsetzt, die mit einer halogenfreien Säure, insbesondere einer aus der Gruppe Schwefelsäure, Phosphorsäure, Oxalsäure oder deren Gemischen, behandelt sind.
